(19) Europäisches Patentamt
European Patent Office
Office européen des brevets

(11) **EP 1 961 432 A1**

(12) **EUROPEAN PATENT APPLICATION**

(43) Date of publication:
**27.08.2008 Bulletin 2008/35**

(51) Int Cl.:
*A61L 27/20* [(2006.01)]    *A61L 27/54* [(2006.01)]
*A61L 31/04* [(2006.01)]    *A61L 31/16* [(2006.01)]
*A61L 33/08* [(2006.01)]

(21) Application number: **07103055.5**

(22) Date of filing: **26.02.2007**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(71) Applicant: **SWETREE TECHNOLOGIES AB
S-90719 Ume (SE)**

(72) Inventors:
  • **Bodin, Aase**
    **43139, Mölndal (SE)**
  • **Gatenholm, Paul**
    **42935, Kullavik (SE)**

  • **Fink, Helen**
    **41323, Göteborg (SE)**
  • **Risberg, Bo**
    **45295, Strömstad (SE)**
  • **Brumer, Harry**
    **12137, Johanneshov (SE)**
  • **Ahrenstedt, Nils Lage**
    **11532, Stockholm (SE)**

(74) Representative: **Linde, Jörgen
    Albihns AB
    P.O. Box 5581
    114 85 Stockholm (SE)**

(54) **Implantable material comprising cellulose and the glycopeptide xyloglucan-GRGDS**

(57)    Implantable materials for medical or surgical applications comprising specific chemical groups on their surface to alter the physico-chemical properties of said material rendering it suitable implantation or biocompatible properties.

**Figure 1.**

EP 1 961 432 A1

**Description**

FIELD OF INVENTION

The present invention relates to implantable materials for medical or surgical applications comprising specific chemical groups that alter the physico-chemical properties of said material rendering it suitable implantation or biocompatible properties. More particularly, the present invention relates to implantable materials comprising polymeric carbohydrates, methods for preparing these implantable materials, as well as the use of materials produced by these methods, and products comprising these materials.

BACKGROUND OF THE INVENTION

Organ or tissue failure is a major health problem. Tissue engineering presents the potential to restore tissue function by using functional healthy cells from different sources (i.e. autogenic, allogeneic, or xenogeneic cells), and/or extracellular natural or synthetic polymers.

A large number of synthetic polymeric materials with various different properties are today used in medical and cosmetic applications like prostheses, implants and scaffold for tissue engineering. These synthetic polymeric materials can generally be divided into two major groups, temporary/bioresorbable and long-term implants/non-bioresorbable. Examples of bioresorbable synthetic materials include polymers comprising poly 1-lactic acid (PLLA) and poly 1-glycolic acid (PLGA). An example of long-term implantable and non-bioresorbable materials is poly(tetrafluoroethylene) PTFE, which has been used in a wide array of medical implantable articles including vascular grafts and tissue repair sheets and patches.

However, these synthetic materials also have limitations and disadvantages such as inadequate interaction between polymer an cells, leading, *in vivo*, to foreign body reactions, such as inflammation, infections, aseptic loosening, local tissue waste, and implant encapsulation as well as thrombosis and embolization. Thus, the success of a promising polymer depends, in part, on the attachment and growth of the cells of interest on its surface. The surface chemistry mediates cellular response to the material and affects cell adhesion, proliferation, migration, and function on the surface.

In the field of arterial vascular reconstructions there is an increasing need for functional small-diameter artificial grafts (inner diameter < 6 mm). When autologous replacement vessels are not available, for example because of the bad condition of the vascular system in the patient, the surgeon has no other alternative than to implant a synthetic polymer-based vessel. After implantation the initial major problem concerning these vessels is the almost immediate occlusion, due to blood coagulation and platelet deposition, under the relatively low flow conditions. Since the interactions that lead to surface induced thrombosis occur at the blood-biomaterial interface, surface modification has been a way to increase hemocompatibility. Among the different modifications, modifications with polysaccharides such as heparin have also been widely used to minimize thrombus formation on artificial organs. A problem however has been that the activity of heparin significantly decreases when it is directly bound to a surface. To date, the most successful type of biocompatible surface has been the end-point-attached heparin surface.

Another attractive surface modification has been to introduce hydrophilic groups that can prevent plasma protein adsorption, platelet adhesion and thrombus formation. The problem has been to get them to remain on the surface for long-term devices such as vascular grafts. Especially, water soluble polymers such as polyacrylamide (PAAm); poly (N, N-dimethylacrylamide) (PDMAAm); poly (ethylene glycol) (PEG), ethylene - vinyl alcohol copolymer (EVA), and poly (2hydroxyethyl methacrylate) (PHEMA) have been grafted onto solid surfaces in order to prevent protein adsorption.

Other ideas for modifying a material to improve its hemocompatibility have to do with mimicking the biologically inert surface. In a blood vessel, this inert surface is composed of a monolayer of endothelial cells. Current materials used as vascular grafts, such as expanded polytetrafluoroethylene (ePTFE) and polyester, do not promote adhesion or proliferation of human endothelial cells. Surface modification has therefore been done with either fibrinogen, fibronectin or with immobilized RGD (Arg-Gly-Asp), which is the minimal fragment of the active site of adhesive proteins such as fibrinogen, fibronectin and von Willbrand factor. Extensive research and trials have been conducted to seed endothelial cells onto PTFE tubes as well as surface modifications with heparin. Problems though are to keep the cells in the long run onto the surface.

However, these synthetic materials mentioned above, e.g. PTFE and polyester, also have other limitations and disadvantages including a limited range of physical and biochemical properties. Thus, there remains a need to explore alternative implantable materials more suitable for use specific surgical applications.

Several investigators have studied tissue biocompatibility of cellulose and its derivatives) as well as examined some specific applications for the material. Specifically, cellulose produced by microorganisms has been investigated for use in tissue engineering. Microbial-derived cellulose has a network structure in which very fine ribbon-shaped fibers composed of highly crystalline and highly uniaxially oriented cellulose are complicatedly entangled with one another, and this network structure contains a large quantity of a liquid in interior voids thereof. Since the cellulose is composed of many ribbon-shaped fibers having a high crystallinity, the cellulose can resist external forces such as a tensile force even in the wet state. The microbial cellulose is not structurally different from cellulose originating from a plant, but a high-order structure such as the above-mentioned structure is not found in the plant-originating cellulose although it is characteristic of the microbial cellulose. Accordingly, the microbial cellulose has a high strength though it is gelatinous.

US 6 800 753 describes the use of regenerated celluloses (RC) and oxidized regenerated celluloses (ORC) for the preparation of scaffold for tissue engineering. The RC and ORC composites are produced by first dissolving cellulose in a solvent system, then regenerating the cellulose into a desired scaffold structure. To produce porous scaffolds, a porogen is introduced in the solvent system to produce pores in the scaffold structure. The scaffold may then be oxidized to introduce carboxyl, aldehyde, and/or ketone functional groups on its surface. These functional groups serve as sites for cell attachment or further chemical modification to induce cell adhesion and subsequent proliferation.

However, the use of regenerated celluloses (RC) and oxidized regenerated celluloses (ORC) produced according to US 6 800 753 poses various problems. For example, it may not be desirable to treat cellulose with solvents because such treatment can be complicated by structural changes to the cellulose material. These structural changes can lead to shrinkage and altered morphology as well as more brittle material.

Currently, as stated above, implantable materials are hampered by problems related to their physical and biochemical properties such as their surface chemistry. Clearly, at present there is a long felt but unmet need to develop new implantable materials and improve their properties so that they can be successfully used in medical applications.

Accordingly, it is a primary objective of the present invention to provide an improved method for the preparation of implantable materials with very good compatibility with the living body, and to provide implantable materials produced with this method.

It is a further objective of the present invention to provide implantable materials with desirable mechanical properties such as mechanical and tensile strength, elongation and sutureability.

Another objective of the present invention is to provide implantable materials provided with attachment sites for cells or other factors affecting cell adhesion, proliferation, migration, and function.

A further object of the present invention is to provide implantable materials that are suitable for *in vivo* implantation.

The method and means of accomplishing each of the above objectives as well as others will become apparent from the description of the invention which follows hereafter.

SUMMARY OF THE INVENTION

In order to fulfil the objects of the present invention, the present inventors provide a novel method for preparing an implantable material, which comprises modifying a polymeric carbohydrate material (PCM) by binding a carbohydrate linker molecule (CLM) comprising a chemical group to the PCM, wherein said chemical group confers improved biocompatibility to the PCM.

One of the major advantages of the method of the present invention for preparing an implantable material comprising a PCM modified with chemical groups, is that chemical treatment of the PCM is avoided. Such treatment can alter the confirmation or orientation of the PCM as well as other physico-chemical properties. Hence, the method of the present invention avoids loss of fibre structure and performance otherwise commonly encountered with direct chemical modification of PCMs.

For example, chemical modification of hydrogels such as bacterial cellulose is complicated by structural changes due to the use of organic solvents. By using an aqueous chemo-enzymatic technique for the surface modification of bacterial cellulose, this invention avoids these structural changes.

In further aspects of the present invention, there are provided implantable materials prepared according to the method of the invention; uses of the implantable material for the manufacture of scaffolds for tissue engineering; scaffolds for tissue engineering comprising a material prepared according to the invention; and methods of in vivo tissue replacement and/or regeneration.

According to a specific embodiment, the present invention encompasses an artificial blood vessel comprising an implantable material prepared according to the invention. The artificial blood vessel of the present invention is characterized by a high penetration resistance, high burst pressure and good biocompatibility.

The present invention will be described in more detail below, inter alia, with reference to the drawings.

BRIEF DESCRIPTION OF THE DRAWINGS

Figure 1 illustrates unmodified polymeric carbohydrate material (PCM) (1), and modified PCM (6). Modified PCM comprises a carbohydrate linker molecule (CLM) (2), said CLM (2) comprising at least a part of a soluble polymeric carbohydrate (SCP) (3), and a chemical group (5) and optionally complexed with a carbohydrate polymer fragment (CPF) (4) comprising the chemical group.

Figure 2 illustrates the preparation of a CLM (2) using an enzyme and a CPF (4). The SCP (8) is contacted with an enzyme (7) and CPF (4) comprising a chemical group (5). The enzyme (7) cleaves the SCP and incorporates the CPF with the chemical group, resulting in the product CLM (2).

Figure 3 illustrates Langmuir adsorption isotherm of bacterial cellulose A and cotton linters • dyed with Direct Red 28 (Congo Red) (A). The line in Figure B shows the results of linear regression.

Figure 4 illustrates Langmuir adsorption isotherm of bacterial cellulose adsorbed with xyloglucan • and xyloglucan-GRGDS ▲ (A). The line in Figure B shows the results of linear regression.

Figure 5 illustrates Langmuir adsorption isotherm of cotton linters adsorbed with xyloglucan • and xyloglucan-GRGDS ▲ (A). The line in Figure B shows the results of linear regression.

Figure 6 shows the adsorbed amount of xyloglucan • and xyloglucan-GRGDS ▲ as a function of specific surface area of the cellulose substrates. The line shows the results of linear regression.

Figure 7 shows crystallinity of bacterial cellulose, cotton linters and lyocell.

Figure 8 shows the chemical structure of the GRGDS xyloglucan oligosaccharide (XGO-GRGDS) (n=1) and XG-GRGDS (n = ca. 30, Mw 32000, Mw/Mn 1.7) glycoconjugates produced by solid-phase synthesis and XET catalysis. Variable glycosylation of the oligo- and polysaccharide is indicated.

Figure 9 illustrates QCM adsorption isotherm of cell culture medium onto cellulose (-), adsorption of xyloglucan onto cellulose (···) followed by cell culture medium. Xyloglucan-GRGDS adsorption onto cellulose (-··) followed by cell culture medium. The arrows represent water wash.

Figure 10 shows light microscopy images of ECs on unmodified bacterial cellulose (A) and xyloglucan-GRGDS modified bacterial cellulose (C). It is clear that the cell adhesion increases and spreading of ECs when bacterial cellulose is modified with an adhesion peptide

Figure 11 shows SEM images of untreated bacterial cellulose (A), bacterial cellulose after treatment in acetone (B) and bacterial cellulose modified with xyloglucan-GRGDS (C).

DETAILED DESCRIPTION OF THE INVENTION

The present invention relates to the development of an implantable polymeric carbohydrate material (PCM) for medical or surgical applications comprising specific chemical groups on their surface to alter the physico-chemical properties of said material. Particularly, said chemical groups confer improved biocompatibility to the PCM, for example by providing attachment sites for cells or other factors affecting cell adhesion, proliferation, migration, and function on the surface.
Furthermore, the present invention relates to the method for preparing the implantable materials of the invention, as well as the use of materials produced by these methods, and products comprising these materials.
In this specification, unless otherwise specified, "a" or "an" means "one or more".
The term "biocompatibility", in relation to the present invention, relates to the property of a material, namely the property of a material to be compatible with the living body. In other words, being harmonious with life; not having toxic or injurious effects on biological function. A material has bad compatibility with the living body if for example an adverse reaction is induced when the material is brought into contact with a part of a living body. This adverse reaction can lead to foreign body reactions, such as inflammation, infections, aseptic loosening, local tissue waste, and implant encapsulation as well as thrombosis and embolization. In contrast, good compatibility with the living body is seen if no such adverse reaction occurs. Furthermore, improved biocompatibility means improved compatibility of a material with the living body. An example of biocompatibility is hemocompatibility, i.e. the property of a material to be compatible with blood.
Throughout the specification, any and all references are specifically incorporated into this patent application by reference.

Method for Preparing Implantable Materials

According to the first aspect of the present invention, there is provided a method for preparing an implantable material by modifying a polymeric carbohydrate material (PCM) by binding a carbohydrate linker molecule (CLM) comprising a chemical group to the PCM, wherein said chemical group confers improved biocompatibility to the PCM.

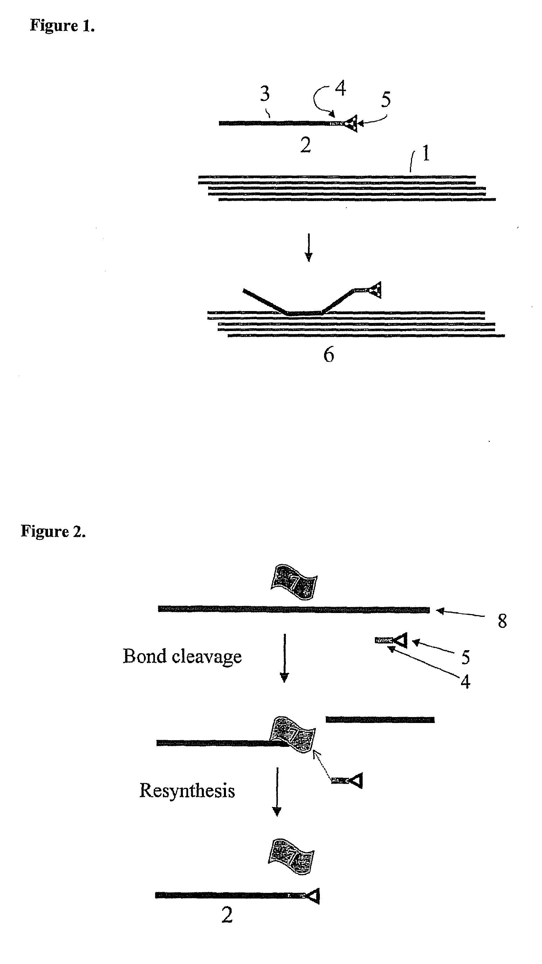

An embodiment of this method is illustrated in Figure 1, showing the unmodified PCM (1), and the carbohydrate linker molecule (CLM) (2), said CLM (2) comprising at least a part of a SCP (3), and a chemical group (5) and optionally complexed with a carbohydrate polymer fragment (CPF) (4) comprising the chemical group. Because the CLM is capable of binding to the PCM, binding will occur when the PCM and the CLM are brought in to contact.

In an embodiment of the invention, the method comprises the steps of: (a) providing a carbohydrate polymer fragment (CPF) comprising a chemical group; (b) bringing said CPF comprising the chemical group into contact with a soluble polymeric carbohydrate (SCP) under conditions leading to the formation of a complex consisting of said CPF comprising the chemical group, and the SCP, said CPF and SCP together forming a carbohydrate linker molecule (CLM); and (c) contacting said complex with the PCM to be modified under conditions where the complex binds to the PCM.

In a preferred embodiment, the step of contacting and binding the CLM comprising a chemical group to the PCM is performed under aqueous conditions. This has the advantage that no changes to the morphology of PCM occur during the step of contacting and binding the CLM to the PCM.

The process of preparing the CLM comprising a chemical group, i.e. the process of preparing a complex consisting of said CLM and said chemical group, is performed separate from the PCM. Thereby, the process of preparing a CLM comprising a chemical group can be upscaled and comprise several steps and harsh conditions.

The term "polymeric carbohydrate materials" which is abbreviated "PCM" relates to a material that comprises a water-insoluble polymeric carbohydrate material and/or a water-soluble polymeric carbohydrate material, which wholly or partly is made up of repeating units of one or more monosaccharides. Such PCMs are often composites with two or more different types of polymeric carbohydrates or a carbohydrate polymer and another polymers such as protein.

The PCM, according to this invention, may be any polymeric carbohydrate material suitable for use as an implantable material, e.g. as the main component of a scaffold for tissue engineering. Different PCMs that can be used in the present invention are, for example, described in WO 03/033 813.

In one specific embodiment, the PCM is in the form of cellulosic material, i.e. comprises cellulose. In the context of the present invention cellulose may be extracted from an annual plant such as for example flax, hemp or cereals or perennial plant such as for example cotton, poplar, birch, willow, eucalyptus, larch, pine or spruce. Examples of appropriate cellulosic materials include purified cotton, cotton linters, $\alpha$-cellulose, wood pulp, purified wood pulp, powdered cellulose, microcrystalline cellulose, and/or cellulose modified to other polymers.

Another source of cellulose that has different properties compared with plant cellulose is microbial-derived cellulose. Microbial-derived cellulose has shown to be interesting in the use as a biomaterial. Mainly because of the possibility to mould it into different shapes for a given application as well as its biocompatibility and high purity. This cellulose is an exopolysaccharide and is produced fairly inexpensive by cultivating *Acetobacter xylinum.* Compared with plant cellulose the cellulose is extruded in its pure form and is not associated with any other polymers or proteins. The bacterial cellulose can be effectively purified with sodium hydroxide achieving endotoxin values in respect to FDA for implants in contact with blood i.e. <20 EU per device. Bacterial cellulose contains 99% of water and can be seen as a hydrogel albeit not by definition. Despite its low solid content, the ramified network of nano-cellulose fibrils provides great mechanics.

The term "microbial-derived cellulose" relates to cellulose produced by a microorganism such as a bacteria, as described above. In one preferred embodiment of the invention, cultures of cellulose synthesizing bacteria such as *Acetobacter xylinum* spp are used.

The term "soluble carbohydrate polymers" which is abbreviated (SCP), relates to polymers comprising one or more different monosaccharides or their derivatives, which can be dissolved in aqueous or organic solvents. Examples include polysaccharides classified as hemicelluloses (those carbohydrate polymers which are not composed only of, $\beta$ (1-4)-linked glucose units, i.e., cellulose), pectins (polyuronic acids and esters), and starches ($\alpha$(1-4)-linked polyglucose with or without $\alpha$(1-6) side chain branching). Xyloglucan, which is a polysaccharide composed of a $\beta$(1-4)-linked polyglucose backbone decorated with $\alpha$(1-6)xylose residues, which themselves can be further substituted with other saccharides such as fucose and arabinose, is an example of such a SCP, specifically a hemicellulose. In a preferred embodiment, the SCP is capable of binding to the PCM, e.g. via one or more hydrogen bonds, ionic interaction, one or more covalent bonds, van der Waals forces or any combination of these. In an embodiment of the present invention, the SCP may be a CPF according to the description below. In a preferred embodiment, the SCP is derived from xyloglucan (XG).

The term "carbohydrate polymer fragments" which is abbreviated "CPF" relates to molecules that may be enzymatically or chemically prepared fragments of the SCPs. Examples of such fragments comprise any number of the repeating units of said SCPs.

Suitable fragments may thus contain from 2 to approximately 5000 monosaccharide units in the polymer backbone such as approximately 2-10, 4-10, 3-100, 11-15, 20-25, 26-40, 41-60, 61-100, 101-200, 201-300, 301- 400, 401-500, 501-1000, 1001-2000, 2001-3000, 3001-4000 or 4001-5000 monosaccharide units. The CPF may further comprise side chains of different length and composition. Specific examples include but are not limited to xylogluco-oligosaccharides (XGO) or a fragment thereof, or as further modified with one or more fucosyl residues or other monosaccharides.

XGOs are commonly named according to the nomenclature system outlined in Fry et al. (1993) Physiologia Plantarum, 89, 1-3 where G represents an unsubstituted beta-glucopyranosyl residue, X represents a xylopyranosyl-alpha(1-6) glucopyranosyl unit, L represents a galactopyranosyl-beta-(1-2)-xylopyranosylalpha(1-6)glucosyl unit, F represents a fucopyranosyl-alpha(1-2)-galactopyranosylbeta(1-2)-xylopyranosyl-alpha-(1-6)-glucosyl unit, among others. These various units may be connected via a beta(1-4) linkage between the glucopyranosyl units to form a beta(1-4)-glucan polysaccharide backbone. Using this nomenclature, the XGOs which are commonly isolated after endoglucanase digestion of tamarind xyloglucan are XXXG, XLXG, XXLG, and XLLG. If the reducing-end glucose (G) of these oligosaccharides is in the reduced, alditol form, this unit is represented by "Gol". Thus, for example, the reduced (alditol) derivatives of the aforementioned oligosaccharides from tamarind xyloglucan are designated XXXGol, XLXGol, XXLGol, and XLLGol.

Preferably, the CPF is derived from xyloglucan and may contain from 3 to about 100 including from 4 to 10 polymer backbone monosaccharide units.

The term "carbohydrate linker molecules" which is abbreviated "CLM", relates to a molecule or complex which contains at least a part of a SCP according to the description above and a chemical group. The CLM is capable of binding to the PCM, e.g. via one or more hydrogen bonds, ionic interaction, one or more covalent 5 bonds, van der Waals forces or any combination of these.

The CLM may be prepared by organic or chemical synthesis and/or by using the catalytic activity of certain enzymes.

In an embodiment of the present invention, the CLM may be prepared using methods described in WO 2004/094646 A1. For example, the CLM comprising a chemical group can be prepared by a method comprising the following steps: preparing xyloglucan fragments from xyloglucan polymers; and attaching one or more chemical groups to the reducing end and/or side chains of the xyloglucan fragments, whereby the CLM comprising a chemical group useful for binding to the PCM is produced.

An embodiment of preparing a CLM using an enzyme and an CPF is illustrated in Figure 2. The SCP (8) is contacted with an enzyme (7) and CPF (4) comprising a chemical group (5). In this embodiment the enzyme (7) cleaves the SCP and incorporates the CPF with the chemical group instead, resulting in the product CLM (2).

The CLM may comprise one or more chemical groups.

In an embodiment of the present invention, the CLM may be prepared using an enzyme capable of transferring native or chemically modified mono- or oligosaccharides onto the ends of oligo- or polysaccharides. Such enzymes include but are not limited to enzymes having high transglycosylation activity but low hydrolytic activity, glucosyl hydrolases with high inherent transglycosylation activity, enzymes which have been engineered to enhance their transglycosylation activity, and glycosyl transferases which use nucleotide sugars as substrates.

Different enzymes that could be used according to the present invention and their properties and how to obtain said enzymes, are described in further detail in WO 03033813.

Specifically, a recently isolated mannan endo-transglycosylase is disclosed in Schroder, R. et al. (PLANTA 224(5): 1091-1102, OCT 2006) and Schroder R. et al. (PLANTA 219 (4):590-600 AUG 2004). Mannan-based polysaccharides are also cell wall components, and presumably have some affinity for cellulose. Mannan transglycosylase is the functional equivalent of XET, but working on galactoglucomannan.

In a preferred embodiment of the present invention the enzyme is a xyloglucan endotransglycosylase (XET, EC 2.4.1.207).

In nature, transglycosylation enzymes, such as the XET enzyme, function in vivo, in the living plant, so the enzyme is clearly able to work in an aqueous environment. The method according to the invention may thus be carried out in an aqueous solution, or it may be carried out in water in the presence of certain components such as a buffer and/or a wetting agent and/or a stabiliser and/or a polymer and/or an organic component reducing the water activity such as DMSO. For further details of these components see WO 03/033 813.

According to a specific embodiment of the present invention, new chemical groups can be added to cellulose materials not containing inherent xyloglucan by first using the XET enzyme to couple the chemically modified XGOs to xyloglucan (XG) in solution followed by sorption of the modified XG onto the cellulose materials.

In the context of the present invention, the term "chemical group" relates to any chemical group of potential interest for modification of the PCM. A Modification of is defined as to alter the functional properties of the PCM. The ability to alter the functional properties of the PCM is, in this sense, inherent in the chemical group. According to the invention, the modification confers improved biocompatibility to the PCM. Hence, the chemical group alters the physical-chemical properties of said PCM rendering it more biocompatible.

Biocompatibility relates to the property of a material to be compatible with the living body. One way of improving the biocompatibility of a material is to influence the adhesion, development, migration, proliferation, differentiation, shape, polarity, and/or metabolic function of cells that come into contact or interact with the material.

Elements included in the chemical group of the invention that alter the physical-chemical properties of the PCM rendering it more biocompatible include but are not limited to: ECM adhesion molecules, growth factors, cell adhesion molecules, and adhesion peptide fragments as well as cell culture substrates and cell nutrients. It would be apparent to an artisan of skill in the art that this list of elements is not exhaustive, and other suitable elements which alter the physical-chemical properties of the PCM rendering it more biocompatible may be used in the present invention.

In relation to the present invention, the term "ECM adhesion molecules" relates to extracellular macromolecules that constitute the extracellular matrix (ECM). These macromolecules, mainly proteins and polysaccharides, are secreted locally and assemble into an organized 3-D meshwork in the extracellular spaces of most tissues. ECM molecules include glycosaminoglycans, and proteoglycans such as chrondroitin sulfate, fibronectin, heparin sulfate, hyaluron, dermatan sulfate, keratin sulfate, laminin, collagen, heparan sulfate proteoglycan, and elastin. Extracellular matrices modulate the organization of the intracellular cytoskeleton, cell differentiation and the spatial architecture of cells and tissues. In fact, the ECM plays a critical role in regulating the behaviour of cells that contact it by influencing cellular development, migration, proliferation, differentiation, shape, polarity and metabolic function.

In relation to the present invention, the term "growth factor" relates to a biologically active polypeptide which causes cell proliferation. These include, without limitation, epidermal growth factor, transforming growth factors, nerve growth factor, acidic and basic fibroblast growth factor and angiogenesis factor, platelet-derived growth factor, insulin and insulin-like growth factors including somatomedins, myxoma and vaccinia virus-derived growth factors.

In relation to the present invention, the term "cell adhesion molecule" relates to cell adhesion molecules that contain cell binding sequences. Examples of cell adhesion molecules include integrins, cadherins, selectins, and adhesion molecules of the immunoglobulin superfamily, such as VCAM, ICAM, PECAM, and NCAM.

The terms "ECM adhesion molecules", "growth factors, or "cell adhesion molecules" include any active analogs, active fragments, or active derivatives thereof.

In relation to the present invention, the term "adhesion peptide fragment" relates to peptide sequences that provide attachment sites for cells or other factors affecting cell adhesion, proliferation, migration, and function on the surface, e.g. peptide sequences improving cell-attachment efficiency.

Several such adhesive peptide fragments are known in the art. A particular peptide fragment can be tested for its binding ability or adhesive capacity according to standard techniques. Examples of such peptide sequences include but are not limited to: RGD-containing peptide sequences; YIGSR-containing peptide sequences; and/or IKVAV-containing peptide sequences.

Arg-Gly-Asp (RGD)-containing peptide sequences are widely recognised as cell recognition motifs. RGD peptides do not only trigger cell adhesion effectively but can also be used to address selectively certain cell lines and elicit specific cell responses. Further details about different RGD-containing peptides that can be used in this invention and there specific properties are described in Hersel et al, Biomaterials 24 (2003) 4385-4415.

Examples of RGD-containing peptide sequences that could be used in the present invention include but are not limited to: RGD, RGDS, GRGDS, GRGD, YRGDS, YRGDG, YGRGD, GRGDSP, GRGDSG, GRGDSY, GRGDSPK, CGRGDSY, GCGYGRGDSPG, and RGDSPASSKP.

In one preferred embodiment of the present invention, the peptide sequence is Gly-Arg-Gly-Asp-Ser (GRGDS).

Tyr-Ile-Gly-Ser-Arg (YIGSR)-containing peptide sequences are found on the B1 chain of laminin, promotes epithelial cell attachment (Graf et al., Biochemistry, 26, pp. 6896-900 (1987)).

Ile-Lys-Val-Ala-Val (IKVAV)-containing peptide sequences are found on the A chain of laminin, and have been reported to promote neurite outgrowth (Tashiro et al., J. Biol. Chem., 264, pp. 16174- 182 (1989.

The chemical group of the present invention can comprise repeating peptide sequences (peptide monomers). The repeating peptide sequences may be homopolymers consisting of a single repeating peptide monomer or alternatively may be heteropolymers consisting of two or more different repeating peptide monomers or subunits. In general the chemical group may consist of about 2 to about 100 peptide monomers, usually about 2 to about 50, preferably about 3 to about 15. Each peptide monomer may range in length from about 2 to about 40 amino acid residues, usually about 2 to about 30, preferably about 2 to about 10.

One of skill will recognize that the peptide monomers may be chemically synthesized or produced by means of recombinant genetics. Similarly, the chemical groups comprising repeating peptide sequences may be produced by chemically linking peptide monomers together or alternatively they can be recombinantly expressed.

In one specific embodiment of the present invention, chemical group of the present invention comprises repeating peptide sequences of RGD-containing peptide sequences.

Scaffold for Tissue Engineering

The implantable material prepared according to the present invention can be used for the manufacture of a scaffold for tissue engineering. One of the advantages of the implantable material of the invention is that it comprises a chemical group that confers improved biocompatibility to the scaffold.

The term "scaffold for tissue engineering" in relation to the present invention relates to a tissue substitute or implant, e.g. a functional replacement of (damaged) tissue.

The present invention offer a wide range of special applications in human and veterinary medicine and in cosmetic surgery, and may be used for any and all indications of previously described scaffolds for tissue engineering, and for other purposes not yet literally disclosed in the art, but readily ascertainable by persons skilled in the art.

Examples of special applications of the implantable material according to the invention include but are not limited to: substitution material or tissue implants for blood vessels (i.e. artificial blood vessels), lymphatic vessels, the uteter, the trachea, the digestive tract, the skin, the oral cavity, the oesophagus, the abdominal wall, the urethra as well as for peridontal tissue, cartilaginous tissue and, subcutaneous tissue. Further applications are protective covers for micronerve sutures; and cultured skin carriers.

The scaffold of the present invention will be specifically shaped for its respective application. Different possible shapes include, but are not limited to, hollow tubes, strips, cylinders, rods and lamina.

According to certain embodiments, microbial-derived cellulose is used as the PCM in the scaffolds of the present invention. Microbial cellulose has many advantageous properties in this regard, for example it can be synthesized in various shapes or sizes with excellent shape retention. These properties of microbial cellulose are mostly attributed to its unique laminar microfibrillar three-dimensional structure. The microfibrils arranged in a non-woven manner are about 200 times finer than plant cellulose such as cotton fibers, yielding tremendous surface area per unit volume. Processes for producing shaped microbial cellulose materials is described in for example JP 8 126 697 A2, EP 186 495, JP 3 165 774 A1 and JP 63 205 109 A1. Furthermore, the production of hollow tube microbial cellulose for use as blood vessel substitutes is described in JP 3 272 772 A2 and EP 396 344 A2.

Scaffolds of the present invention can in addition to the implantable material according to the present invention also comprise auxiliary materials. Appropriate auxiliary materials for this purpose include water-soluble, polar solvent-soluble or hydrophilic gel-forming polymeric materials such as agar, dextran, polyacrylamide, polyvinyl pyrrolidone, alginic acid salts, hyaluronic acid, curdlan, polyacrylic acid salts, heparin, sulfated polysaccharides, pullulan, carrageenan, glucomannan, cellulose derivatives, polyethylene glycol, polyvinyl alcohol, gelatin, collagen, laminitol, fibronectin, keratin, silk hydrolyzate, polyamino acids, poly-organic acids and enzymes. The implantable material according to the present invention is combined with an auxiliary material as mentioned above by means such as impregnation, lamination or adsorption to obtain a composite.

According to certain embodiments, scaffolds of the present invention comprise a PCM derivatized with chemical groups comprising elements that influence the adhesion, development, migration, proliferation, differentiation, shape, polarity, and/or metabolic function of cells that come into contact or interact with the derivatized PCM.

Thus, according to the compositions and methods of this invention, it may be possible to influence the behaviour (i.e., adhesion development, migration, proliferation, differentiation, shape, polarity, and/or metabolic function) of any cell type, by providing the appropriate molecular motifs.

Particularly, chemical groups comprising elements that influence cell adhesion are used according to the present invention.

The PCM of this invention can be used to present cell adhesion molecules, or adhesion peptide fragments, to a variety of cell types. These cell types include any cell that is normally in contact with the implanted material *in vivo*. Such cells include but are not limited to epithelial cells, endothelial cells, fibroblasts, myoblasts, chondroblasts, osteoblasts, and stem cells. Other cells that may be useful in the methods and products of this invention include, Schwann cells, astrocytes, oligodendrocytes and their precursors, adrenal chromaffin cells, and the like.

Stem cells represent a class of cells which may readily be expanded in culture, and whose progeny may be terminally differentiated by the administration of a specific growth factor. Myoblasts are muscle precursor cells originally derived from mesodermal stem cell populations, e.g., L-6 and O-CH3 cells.

It will be appreciated that the choice of chemical group for use in the invention can for example depend upon the desired target cell type. One of skill in the art can routinely assay any particular cell adhesion molecule or adhesion peptide fragment motif for its adhesive capacity for a chosen cell type.

In other embodiments, scaffolds of this invention can be pre-seeded with cells *in vitro,* whereby the cells are exposed to the PCM. Functional healthy cells from different sources (i.e. autogenic, allogeneic, or xenogeneic cells) can be used. These cell-seeded scaffolds are useful in tissue replacement protocols. According to these embodiments, tissue can be reconstituted *in vitro* and then implanted into a host in need thereof. For example, cardiac myoblasts may be suspended in the scaffold of this invention to create a tissue patch of a thickness corresponding to the cardiac wall. The reconstituted cardiac patch could then be implanted, as part of a tissue replacement therapy. Similar protocols for blood vessels, cartilage, tendon, bone, skin, nerve, and other tissues are contemplated.

With the use of the method of the invention, it is possible to modify the implantable material, i.e. the scaffold, at the site of interest. For example, it would be possible to just modify one side of a sheet or tube of a scaffold. The density of the PCM as well as the length of the CLM will influence if the entire network of the PCM or just one side will be modified. Hence, it is possible to optimize the extent of modification of the PCM and the site of modification, by changing the density of the PCM or the length of the CLM and thereby changing the accessibility of the CLM to penetrate and adsorb. According to this embodiment, the present invention renders it possible to modify at a specific site and thereby giving dual functionality to the scaffold/implant. For example, the inner wall of a artificial blood vessel can be modified with chemical groups that promote adhesion or proliferation of human endothelial cells whereas the outer wall of the same artificial blood vessel can be modified with chemical groups that promote the biocompatibility with the surrounding tissue of the blood vessel.

The scaffolds of this invention may be further modified by preincubating the PCM in cell culture media prior to cell seeding onto the material. This is done to improve the adhesion of cells to the material.

In certain embodiments, the scaffold of this invention may be used in the preparation of an artificial blood vessels. Processes for obtaining artificial blood vessels comprising PCM are well known in the art. Artificial blood vessels of this invention can be of any dimension, linear, tapered and/or branched.

In accordance with a preferred embodiment of the present invention, the artificial blood vessel comprises microbial-derived cellulose. EP 0 396 344 and JP 3 272 772 describe processes for obtaining artificial blood vessels comprising microbial-derived cellulose. The microbial cellulose can for example be obtained by culturing a cellulose-producing microorganism on the inner surface and/or outer surface of a carrier composed of, e.g., cellophane, Teflon, silicon, ceramics, a non-woven fabric or a woven fabric.

Bodin et al., Influence of cultivation conditions on mechanical and morphological properties of bacterial cellulose tubes, Biotechnol Bioeng, 2006 Dec 29 (Epub ahead of print), describes an improved method for obtaining artificial blood vessels comprising microbial-derived cellulose. According to this method, bacterial cellulose was deposit in tubular form by fermenting *Acetobacter xylinum* on top of silicone tubes as an oxygenated support and by blowing different concentrations of oxygen, i.e. 21% (air), 35%, 50% and 100%.

Furthermore, the microbial cellulose of the artificial blood vessel is modified, in accordance with the method of the invention, by the binding of a chemical group which confers improved biocompatibility and hemocompatibility to the microbial cellulose as described above. The improved biocompatibility and hemocompatibility of the artificial blood vessel of the invention greatly decreases the risk for problems like occlusion. Occlusion is a major problem and is common under the relatively low flow conditions of small diameter artificial blood vessels. Occlusion is caused by blood coagulation and platelet deposition. Since the interactions that lead to these problems occur at the blood-implant interface, modification according to the invention is a way to increase hemocompatibility and decrease problems with occlusion.

The artificial blood vessel of the present invention is further characterized by a high penetration resistance and high burst pressure.

In a preferred embodiment of the invention, the implantable material of the artificial blood vessel comprises chemical groups comprising at least one RGD-containing peptide sequence. These sequences will promote adhesion or proliferation of human endothelial cells to the blood vessel wall.

Furthermore, the artificial blood vessels can be pre-seeded with endothelial cells prior to implantation.

Tissue Replacement and Regeneration

The scaffolds comprising implantable material according to the present invention have various medical or surgical applications.

According to a further aspect of the present invention, there is provided a method of in vivo tissue replacement and/or regeneration comprising the following steps: a) providing a scaffold for tissue engineering comprising implantable material prepared according to the present invention; and b) implanting said material into a suitable implantation site of a subject in need thereof.

The terms "tissue replacement" and "tissue regeneration" in relation to the present invention refer to tissue engineering in general and deals with the functional replacement of damaged tissue and tissue regeneration. The scaffold comprising implantable material of the invention can act as tissue replacement, whereby the host tissue is completely or partially replaced by the implanted material. Further, the scaffold comprising implantable material of the invention can act as a support for tissue regeneration, whereby the host cells infiltrate the material. According to this embodiment, host tissue regeneration is facilitated.

The procedures used for implanting the scaffolds of the invention are those generally used for tissue engineering in for example general surgery, plastic surgery or neurosurgery. These procedure is adopted according to subject and implantation site. Implantation site could in principle be any part of the living body, e.g. the vascular system, the lymphatic system, the skin, the nerve system, etc. Subjects according to the invention could be any subject in need of tissue replacement e.g., a mammal, preferably a human.

In a specific embodiment, the scaffold is pre-seeded with cells *in vitro* before the step of implanting said scaffold.

In a further embodiment, the scaffold for tissue engineering is an artificial blood vessel.

The following examples are given to illustrate the present invention. It should be understood, however, that the invention is not intended to be limited to the specific conditions and details described in these examples.

EXAMPLES

The present inventors surface modified bacterial cellulose with xyloglucan-GRGDS conjugates, and analyzed the effects of GRGDS modified bacterial cellulose on endothelial cell adhesion.

Materials

Bacterial cellulose (BC) hydrogel and Whatmann filter paper, Grade 1, made from pure cotton linter used for comparison, were investigated in adsorption studies of Congo Red and xyloglucan. Trimethylsilyl cellulose (TMSC) was used in the adsorption study using QCM. The TMSC was synthesized as described in Kontturi et al, Langmuir 19 (2003) 5735-5741. BC was grown statically in a corn steep liquid medium, using Roux flasks (working volumes 100ml) at 30°C for three days, giving a pellicle of 2mm. The strain used for the biosynthesis was *Acetobacter xylinum* subsp. *sucrofermentas* BPR2001, trade number: 1700178™. The strain was purchased from the American Type Culture Collection. The BC was purified by boiling in 0.1 M NaOH, 60°C, for four hours and thereafter repeated boiling in Millipore™ water. The material was steam sterilized and stored rerfrigerated before use. Acetone treatment: Bacterial cellulose was treated with acetone over night prior to freeze drying and SEM analysis.

EXAMPLE 1

Preparation of Xyloglucan and Xyloglucan-GRGDS

A mixture (15:7:32:46) of XXXG, XLXG, XXLG and XLLG xylogluco-oligosaccharides (XGOs) was obtained by direct treatment of tamarind kernel powder (60% xyloglucan content, D.N. Palani, Mumbai, India) by *endo*-glucanase digestion, essentially as previously described in Greffe, L., et al., Glycobiology, 2005. 15(4): p. 437-445. The XGOs were activated by conversion to the corresponding 1-deoxy-1-aminosuccinamate derivatives (XGO-succ) via the 1-deoxy-1-amino-β-glycosides as follows. XGO (1 g, 0.78 mmol) was dissolved in deionised water (10 mL) followed by addition of ammonium hydrogen carbonate (2.5 g), the mixture was then stirred at 42° C for 28 h with continuous addition of ammonium hydrogen carbonate to maintain saturation. The progress of the reaction was followed by TLC (70/30 acetonitrile/water). Excess ammonium hydrogen carbonate was removed by three cycles of freeze-drying to yield a white powder consisting of a mixture of XGOs and 1-deoxy-1-amino-β-glycosides; the extent of conversion was 83%, based on integration of $^1$H-NMR signals from the anomeric protons of the starting material and product. The crude product was dissolved in water (10 mL), succinic anhydride (157 mg, 1.57 mmol, 2 equiv.) was added, and the solution was vigorously stirred on vortex mixer for 10 minutes. Reversed-phase (C 18 silica gel) chromatography using step-wise elution with water/acetonitrile mixtures containing 0.1 % TFA yielded XGO-succinamate as a white powder (860mg, 0.63 mmol, 80% yield over two steps). $^1$H NMR (500 MHz, $D_2O$, 25 °C): δ= 2.56 (t, J = 6 Hz, 2H; COC$H_2$CH$_2$COOH), 2.61 (t, J = 7 Hz, 2H; COCH$_2$C$H_2$COOH), 3.24-3.95 (m; H-2 to H-6 of Gal, Glc, 1-deoxy-1-aminosuccinate-Glc and H-2 to H-5 of Xyl), 4.44-4.50 (m; H-1 of Glc and Gal), 4.85-4.89 (m; H-1 ofXyl), 5.08-5.10 (m; H-1 ofXyl bearing Gal-beta-(1-2)). ESI-MS [37]: XXXG-succ [M+2Na]$^{2+}$, 603.6802 calculated (603.7095 observed); XLXG-succ and XXLG-succ [M+2Na]$^{2+}$, 684.7066 calc. (684.7079 obs.); XLLG-succ [M+2Na]$^{2+}$, 765.7330 calc. (765.7475 obs.).

The pentapeptide GRGDS was synthesised on a 0.25 mmol scale using standard solid-phase Fmoc chemistry according to the protocol described by Engfeldt et al., Chembiochem: a European journal of chemical biology, 2005. 6 (6): p. 1043-50, with the following exceptions. The Fmoc protected amino acids were activated with HBTU and HOBt (both 0.45 M in DMF) in presence of DIPEA (2.0 M in NMP). Capping steps were excluded. Following the final Fmoc cleavage step, the peptide substitution of the resin was determined to be 0.47 mmol/g. XGO-succ was manually conjugated to the resin-bound peptide in a reaction vessel equipped with a glass frit filter (pore size P2). XGO-succ (260 mg, 2 equiv.) was dissolved in DMF (6 mL) and activated with HBTU (215 mg, 6 equiv) and HOBt (87 mg, 6 equiv) in the presence of DIPEA (66 mL, 4 equiv). The resin bound peptide (200 mg, 1 equiv) was then added. The coupling was terminated after 1 h by extensive washing of the resin with ethanol, NMP, DIPEA (5% in DCM), NMP and DCM (10 mL each). The resin was subsequently dried under vacuum. The glycopeptide was cleaved from the resin with simulaneous removal of the side-chain protection groups with 3 mL TFA/$H_2O$/TIS (95:2.5:2:5) for 30 minutes at room temperature. The reaction was diluted with water (40 mL), extracted with tBME (3 x 40 mL) and filtered through glass fibres. Freeze-drying the aqueous phase yielded a white solid (82 mg, 47% yield). Under identical conditions, cleavage and de-protection of the unmodified peptide from 75 mg of resin yielded 15 mg of GRGDS (90% yield).

GRGDS: $^1$H NMR (500 MHz, $D_2O$, 25 °C): δ= 1.52-1.81 (m, 4H; 2 H$^\beta$-Arg, 2H$^\chi$-Arg), 2.65 (d, J = 6.5 Hz, 2H; H$^\beta$-Asp), 3.16 (t, J = 7 Hz, 2H; H$^\delta$-Arg), 3.80-3.92 (m, 6H; 4 H$^\alpha$-Gly, 2 H$^\beta$-Ser), 4.26 (t, J = 7 Hz, 1H; H$^\alpha$-Arg), 4.35 (t, J = 5 Hz, 1H; H$^\alpha$-Ser), 4.60 (t, J = 6.5 Hz, 1H; H$^\alpha$-Asp). ESI-MS: [M+H]$^+$ 490.2376 calc. (490.2109 obs.)

XGO-succ-GRGDS: δ= 1.53-1.86 (m, 4H; 2 H$^\beta$-Arg, 2H$^\varkappa$-Arg), 2.55-2.60 (m, 2H; H$^\beta$-Asp), 2.80-2.92 (m, 4H; XGO-NH-COC$H_2CH_2$CO-Gly), 3.12-3.16 (m, 2H; H$^\delta$-Arg), 3.25-3.98 (m: H$^\alpha$-Gly, H$^\beta$-Ser, H-2 to H-6 of Gal, Glc, 1-deoxy-1-aminosuccinate-Glc and H-2 to H-5 of Xyl), 4.25-4.30 (m, 1H; H$^\alpha$-Arg), 4.34-4.36 (m, 1H; H$^\alpha$-Ser), 4.68-4.74 (m, H-1 of Glc and Gal), 4.87-4.91 (m, H-1 ofXyl), 5.09-5.11 (m, H-1 of Xyl bearing Gal- (1-2)). ESI-MS: XXXG-succ-GRGDS [M+H+Na]$^{2+}$, 828.2988 calc. (828.3080 obs.); XXLG-succ-GRGDS and XLXG-succ-GRGDS [M+H+2Na]$^{3+}$ 613.8800 calc. (613.8911 obs.), [M+H+Na]$^{2+}$ 909.3252 calc. (909.3289 obs.); XLLG-succ-GRGDS [M+H+2Na]$^{3+}$, 667.8976 calc. (667.9045 obs.), [M+3Na]$^{3+}$ 675.2249 calc. (675.2198 obs.), [M+H+Na]$^{2+}$ 990.3516 calc. (990.3554 obs.).

The final xyloglucan-GRGDS glycoconjugate was prepared using xyloglucan *endo*-transglycosylase (XET)-mediated coupling as follows. Tamarind xyloglucan (Megazyme, Ireland) was dissolved in water (2 mg/mL) and 200 mL was mixed with XGO-succ-GRGDS (100 mL, 2 mg/mL in H$_2$O), H$_2$O (50 mL), and a solution of the *Ptt*XET16A enzyme (0.4 units/mL, 50 mL in 100 mM NaOAc, pH 5.5). After 35 min, the reaction was terminated by heating the solution to 85° C for 1 hour. Denatured enzyme was removed by filtration on a glass fiber filter and the product was precipitated from the filtrate by the addition of ethanol (3 vol). The precipitate was collected on a glass fiber filter and redissolved by stirring and gentle heating of the filter in water (20 mL). The resulting solution was freeze-dried to yield 390 mg of XG-GRGDS. Analysis by HP-SEC in DMSO indicated that the product had a $M_w$ value of 32000 ($M_w/M_n$ 1.7). Unmodified xyloglucan with a similar molecular mass ($M_w$ 36000 ($M_w/M_n$ 1.5) was produced using the same procedure, except that XGOs were substituted for XGO-succ-GRGDS.

Alternative Route for the Preparation of Xyloglucan and Xyloglucan-GRGDS

Tamarind kernel powder TKP (Xyloglucan, Mw 500 000 - 1 500 000) was digested with cellulase to form low molecular weight xyloglucan (low Mw XG), Mw 5000-50 000. Prior to linkage of GRGDS peptide to the low Mw XG, the low Mw XG was activated with succinate (see description above). The GRGDS peptides were synthesised with standard solid-phase Fmoc chemistry, and linked to the succinated low Mw XG according to the description above. These GRGDS peptide-low Mw XG can be used directly to modify the bacterial-cellulose.

EXAMPLE 2

Adsorption of Congo red

The specific surface area of bacterial cellulose and cotton as a reference was evaluated by determining the maximum amount of adsorbed Congo Red dye (Direct Red 28, Purchased from Riedel-de Haën, Germany). Six Whatman papers No. 1 and 6 bacterial cellulose gels were used at each adsorption concentration. The cellulose materials were exposed to Congo Red in 4ml of aqueous solution containing 0.5, 1.5, 2.0, 2.5, 3.0, 3.5, 4.0, 4.5, 5.0 (w/w) of Congo red and were dyed for 24 hours at 60°C with a liquid ratio of 100:1. NaCl (20% w/w) was added as an electrolyte. The residual concentration [E, mg/ml] of Congo Red was calculated from the UV adsorption at 492nm using a standard curve. The adsorbed amount of Congo red on fibre [A, mg/g] was calculated from the difference in adsorption at 492nm of the solution before and after the binding reaction, divided by the mass of fibre per volume of solution

EXAMPLE 3

Adsorption of Xyloglucan (XG) and Xyloglucan-GRGDS (XG-GRGDS)

The specific surface areas of bacterial cellulose and cotton linters as a reference was evaluated by determining the maximum amount of XG and XG-GRGDS adsorbed. Six Whatman papers No. 1 and 6 bacterial cellulose gels were used at each adsorption concentration. The cellulose materials were immersed in 4ml of aqueous solution containing 5, 10, 15, 20% (w/w) of xyloglucan or xyloglucan-GRGDS. The XG adsorbed was measured by the colorimetric method described by Kooiman et al., (Recueil des Travaux Chimiques des Pays-Bas et de la Belgique 79 (1960) 675-678. 200$\mu$l was withdrawn at various time intervals from 0 to 48h and mixed with 1ml of a 5:1 solution of 20% (w/v) Na$_2$SO$_4$ and triiodide solution (0.5% I$_2$ + 1% KI). The residual concentration [E, mg/ml] of XG was calculated from the adsorption at 620nm using a standard curve. The amount of XG adsorbed on fibre [A, mg/g] was calculated from the difference in adsorption at 620nm of the solution before and after the binding reaction, divided by the mass of fibre per volume of solution.

EXAMPLE 4

Specific surface area

The specific surface area of cotton linters and bacterial cellulose derived from adsorption of Congo Red versus xyloglucan and xyloglucan-GRGDS was calculated using equation 1 derived from Langmuir's adsorption theory:

$$\frac{[E]}{[A]} = \frac{1}{K_{ads}[A]_{max}} + \frac{[E]}{[A]_{max}} \qquad (1)$$

where [E] is (mg/ml) the concentration of adsorbate at adsorption equilibrium, [A] (mg/g cellulose sample) the amount of adsorbate adsorbed to the cellulose surface, [$A_{max}$] (mg/g cellulose sample) the maximum amount of adsorbate adsorbed to the cellulose surface and $K_{ads}$ the adsorption equilibrium constant. The specific surface, $A_{sp}$, is expressed as:

$$A_{sp} = \frac{[A]_{max} N_A A_{CR}}{10^{21} Mw} \qquad (2)$$

where Mw is the molecular weight of Congo Red (653 g/mol) ;xyloglucan (36 000 g/mol); xyloglucan-GRGDS (32 000 g/mol), $N_A$ is Avogadro's constant and $A_{CR}$ is the area occupied by one Congo Red (1.73 nm$^2$); xyloglucan (69 nm$^2$); xyloglucan-GRGDS (61 nm$^2$). Values for Congo Red were calculated by Ougiya et al., Bioscience, Biotechnology, and Biochemistry 62 (1998) 1714-1719. Values for xyloglucan and xyloglucan-GRGDS were derived by presuming a linear decrease in the occupied area of a xyloglucan with molecular weight, i.e. extrapolating Ougiya *et al*. Values of $A_{CR}$ 1870 nm$^2$ for high molecular weight xyloglucan (980 000g/mol) to lower molecular weight xyloglucan, i.e. 32 000 and 36 000 g/mol.

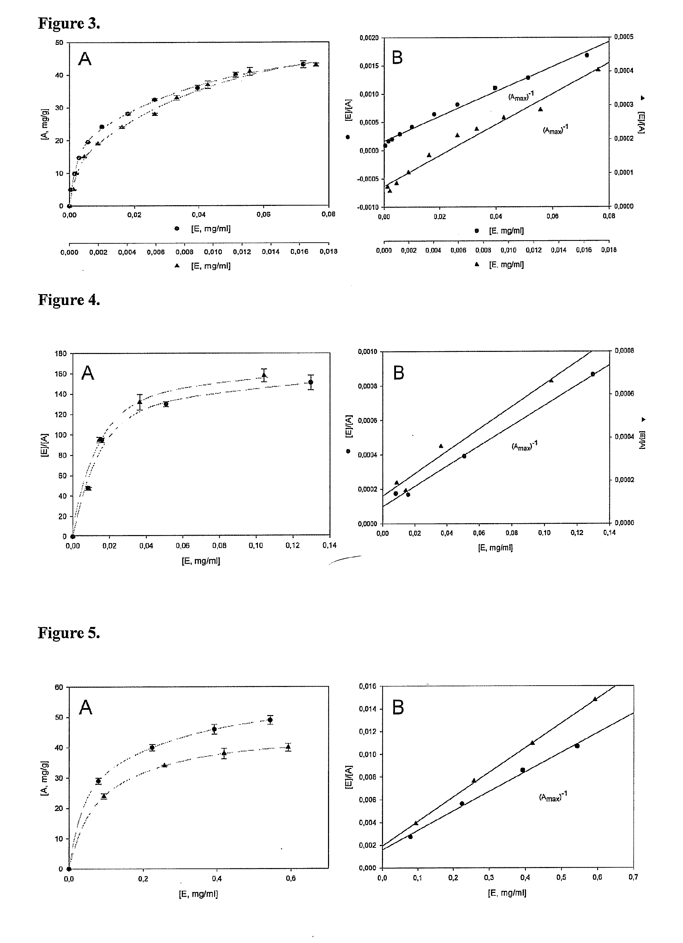

As shown in Figure 3B, a straight line was obtained from equation (1) suggesting that the adsorption of Congo Red onto both substrates followed the Langmuir model. The Congo Red thus most likely adsorbs as a monolayer onto both cellulose surfaces. The adsorption maximum ($A_{max}$) calculated from the slope value reached 47mg/g for both surfaces. The specific surface area for BC (79m$^2$/g) is more or less the same as that of cotton linters (72m$^2$/g), see equation (2). The specific surface area of cotton linters corresponds quite well to values found in the literature for dye adsorption studies while the specific surface of BC was somewhat lower than earlier reported values. This difference was expected however, as the BC in this case was not disintegrated and most likely had a less exposed surface for adsorption.

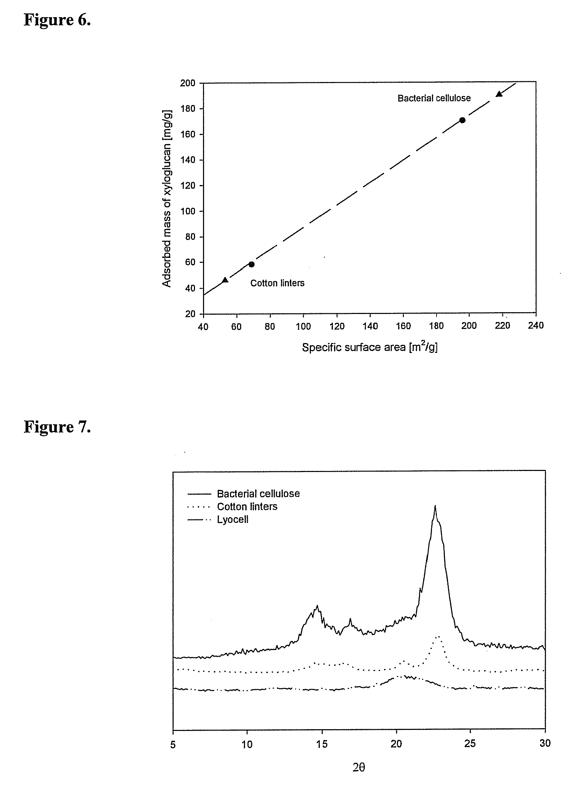

Xyloglucan and xyloglucan-GRGDS also followed a Langmuir adsorption behaviour, see Figure 4B and 5B. The adsorption maximum ($A_{max}$) of XG and XG-GRGDS reached around 180mg/g on BC and only about three times as much on cotton linters, see Figures 4A and 5A. The specific surface area of BC measured with xyloglucan and xyloglucan-GRGDS was around 200m$^2$/g and was almost three times less for cotton linters, 60 m$^2$/g. Both cellulose surfaces conform to a linear relationship with larger surface areas corresponding to higher amounts of absorbed xyloglucans, see Figure 6.

The difference in the amount of adsorbed xyloglucans can probably be explained by the swollen network of bacterial cellulose and a more exposed and accessible bulk compared to cotton linters. It is known that the size of the adsorbate molecule has a significant influence on the accessible surface area, which leads to the conclusion that less of the cotton surface is available for adsorption of xyloglucans. The Congo Red molecule is about 2.5nm in length along its longitudinal axis, while a fully extended xyloglucan backbone with DP 26 is around 30nm. The difference in the specific surface of xyloglucan onto the two cellulose substrates might also be due to differences in crystalline structure. Bacterial cellulose and cotton linters have the same crystal structure, i.e. cellulose I, see Figure 7, and the relatively crystallinity is 70% for both substrates. The material however have different amounts of the crystalline sub-allomorphs (Iα or Iβ), being 60% Iα: 40% Iβ in BC and only 30% Iα : 70% Iβ in cotton linters. This may influence the physical properties of the cellulose as the allomorphs have different crystal packing, molecular conformation and hydrogen bonding.

EXAMPLE 5

Scanning Electron Microscopy (SEM)

SEM was used to study the surface morphology of the unmodified and modified cellulose materials. The bacterial cellulose materials were quenched in liquid nitrogen prior to freeze drying. The surfaces were then coated with gold before analysis, which was done with a Zeiss DSM 940A operated at 10kV.

EXAMPLE 6

Confocal laser microscopy

Confocal microscopy equipped with a fibre coupled ArKr laser was used to study the morphology of bacterial cellulose in its wet state and the modification throughout the gel. Filters were chosen with regard to the emission wave length of the dye [$\lambda_{ex}$=495nm and $\lambda_{em}$=516]. The wet bacterial cellulose samples were fluorescently labelled by a fluorescently labelled xyloglucan (XG-FITC). XG-FITC, was synthesized as described in Brumer, H., et al., Journal of the American Chemical Society, 2004. 126(18): p. 5715-5721. The wet gel was stained for 24 hours with a stock solution of 2mg/ml XG-FITC. After staining, excess XG-FITC was removed by placing the samples in deionised water with mild stirring overnight.

EXAMPLE 7

ESCA

The chemical composition of bacterial cellulose was determined with ESCA before and after surface modification with xyloglucans. The materials were oven dried at 30 °C after modification and prior to the measurements. A Quantum 2000 from Physical Electronics was used for the measurements. The area analysed was 500x500$\mu$m2 and the beam size was 100 $\mu$m. The angle between the sample and detector was 45°C. The peak intensities were measured and curve fitting was done using the MultiPak software from Physical Electronics. Characteristic ESCA spectra of cellulose has one peak at 286.7eV corresponding to carbon single-bonded to oxygen and one at 287.9eV corresponding to carbon bonded to two oxygen. The relative amounts of different bound carbon were calculated with Gaussian curve fitting of the highest resolution Cls peak. The different positions of the C-C, C-O, O-C-O or C=O and O-C=O were 285.0 $\pm$0.2 eV, 286.7 $\pm$0.2 eV, 281.1 $\pm$0.2 eV and 289.4 $\pm$0.2 eV, respectively.
ESCA shows that the surface has been modified with xyloglucans. A slight increase in the amount of carbons bounded to oxygen from the side groups of xyloglucans can be seen. It is impossible to quantify the amount of GRGDS, however, probably as the size of the group is small and might be embedded and pointing towards the interior of the gel when oven dried. There are also traces of nitrogen in the xyloglucan and cellulose itself, which complicate the characterization further.

EXAMPLE 8

Dynamic contact angle towards water

Static contact angle measurements were made on six oven dried cellulose films, unmodified as well as modified cellulose with XG or XG-GRGDS. A 5$\mu$l liquid droplet was applied on each cellulose surface. The contact angle, $\theta_e$, was measured using a goniometer by registering the angle formed between the solid and the tangent to the drop surface.
The wettability is somewhat higher when modified with xyloglucan and xyloglucan-GRGDS as compared to unmodified bacterial cellulose (Table 1).

Table 1

| Surface | Contact angle, $\theta_e \pm$ SD |
|---|---|
| Bacterial cellulose | 44 $\pm$ 5.3 |
| Bacterial cellulose + XG | 29 $\pm$ 4.8 |
| Bacterial cellulose + XG-GRGDS | 32 $\pm$ 5.8 |

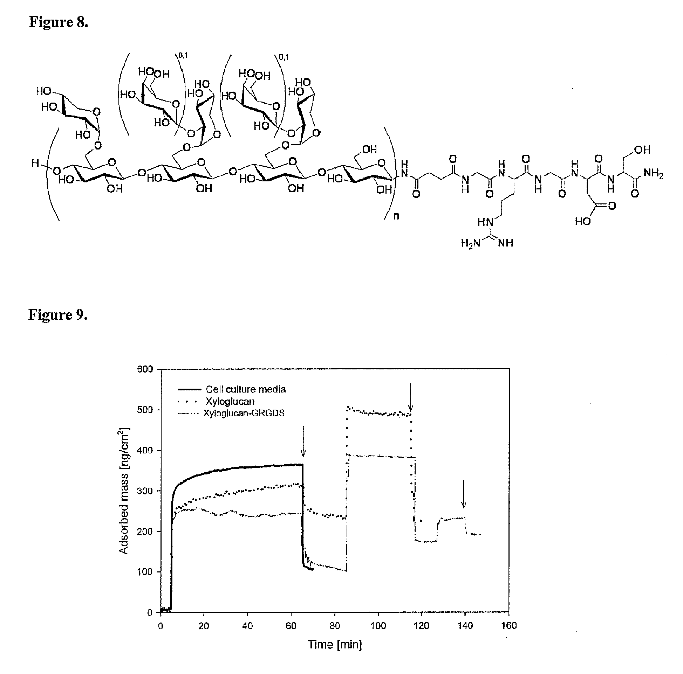

The relatively high contact angle towards water for unmodified bacterial cellulose is due to the compact structure, few pores for capillary forces, and few available hydroxyl groups because of the high crystallinity. Modification with xyloglucans increases the available hydroxyl groups and thereby increases the wettability. Introducing a GRGDS did not lower the wettability significantly, see the structure in Figure 8. Still, the wettability is higher compared to unmodified BC.

EXAMPLE 9

Protein Adsorption using QCM

A QCM-D instrument was used (Q-sense AB, Göteborg, Sweden) to study the adsorption of proteins to the cellulose surface as an effect of surface modification. The model cellulose surfaces were prepared on gold plated QCM-D crystals. Surfaces were cleaned in an UV/Ozone chamber for 10min, followed by immersion in a 5:1:1 mixture of Milli-Q water, $H_2O_2$ (30%) and $NH_3$ (25%) for 10min at 70°C. The surfaces were washed with Milli-Q and dried with nitrogen. Thrimethylsilyl cellulose (1 mg/ml in toluene) was spin coated onto the gold surfaces at 4000rpm, 1min. The thrimethylsilyl groups were cleaved away and cellulose was generated over hydrochloric vapor (10% solution). Measurements were made at the third overtone (15Hz). A change in f reflects the amount of mass coupled to the surface of the crystal. For thin, evenly distributed rigid films, an adsorption induced frequency shift ($\Delta$f) is related to the mass uptake as described with the Sauerbrey equation:

$$\frac{m}{A} = \frac{Cf}{n_r}$$

where $m$ is the mass (ng), A is the area (cm$^2$), $n_r$ is the overtone number (=1,3...) and $C$ is the mass sensitivity constant (17.7 ng/cm$^2$/Hz). Measurements were made in triplicate. Xyloglucan and xyloglucan-GRGDS were adsorbed at a concentration of 2mg/ml. After each adsorption followed a desorption step with water after the cell culture medium was introduced. The same culture medium used in cell seeding was used to study protein adsorption. The cell culture medium contains a mixture of proteins including the cell adhesion protein fibronectin bearing a RGD motif. To elucidate whether the modification with XG-GRGDS resulted in an increase in protein adsorption (and particularly in fibronectin) from the cell culture medium, adsorption studies were done on model cellulose surfaces using QCM-D. Antibody against fibronectin (Fibronectin antibody (Biotin) (ab6584), Abcam) was introduced after cell culture medium was introduced in order to verify whether any of the possibly adsorbed proteins were fibronectin. After all adsorptions followed a desorption step with water.

As shown in Figure 9, around 100 ng/cm$^2$ of proteins from the cell culture medium was adsorbed to unmodified cellulose surface. When xyloglucan was first adsorbed no proteins were adsorbed. When the cellulose was modified with xyloglucan bearing the adhesion pentapeptide less protein was adsorbed (50ng/cm$^2$) compared to unmodified cellulose. Antibody IgG against fibronectin was introduced after modification with xyloglucan-GRGDS and cell culture medium. IgG against fibronectin did not adsorb, which indicates that the proteins adsorbed are not the adhesion protein fibronectin, or at least not in an activated form.

EXAMPLE 10

Wide angle X-ray Scattering (WAXS)

Freeze dried pellets of BC were pressed to pellets having a diameter of 1 cm. X-ray diffraction patterns were recorded on a Siemens D5000 diffractometer. A CuK$\alpha$ anode with a wavelength of 1.54Å was used. The scanning was made through 2$\theta$ = 5-30°. The intensity of the crystal diffraction peak of the amorphous diffraction was measured. The relative crystallinity was determined as the ratio between the crystal part and the total part.

EXAMPLE 11

Cell Seeding

Endothelial cells (HSVECs) were isolated from healthy parts of human saphenous veins using an enzymatic method. Cells were cultured in M199 (PAA Laboratories GmbH, Linz, Austria) supplemented with 20% fetal bovine serum (FBS; PAA Laboratories GmbH) with 1.7 - 3.4 g/dl albumin and a total protein content of 3 - 4.5g/ml, penicillin-streptomycin (100 U/mL; PAA Laboratories GmbH), 1.2 mM L-glutamine (PAA), bovine brain extract (75mg/500mL;

prepared in the laboratory) and heparin (13U/mL; Leo Pharma, Malmö, Sweden) and kept at 37°C in a humidified incubator with 5% $CO_2$. Prior to cell seeding BC was modified with xyloglucan and XG-GRGDS corresponding to 15% of the dry weight of BC overnight. The modified cellulose was washed 2x with PBS before cell seeding.

For assessment of cell morphology, HSVECs were seeded on pieces of modified and unmodified BC at a density of 3 x $10^5$ cells/cm$^2$. Samples were taken out for evaluation at day 1 and day 3. Cells were fixed in 3.7% formaldehyde and permeabilized in 0.2% Triton X-100. To visualize f-actin, cells were stained with phalloidin conjugated to Alexa Fluor® 546 (Molecular Probes Inc., Eugene, OR, USA). The nuclei were counterstained with DAPI (Sigma-Aldrich Sweden AB, Stockholm, Sweden). The specimens were mounted in Slowfade™ Antifade mounting medium (Molecular Probes Inc.) and analyzed with an Axio Imager M1 (Carl Zeiss, Göttingen, Germany). Pictures were captured digitally with an AxioCam HRc (Zeiss).

Cell Adhesion

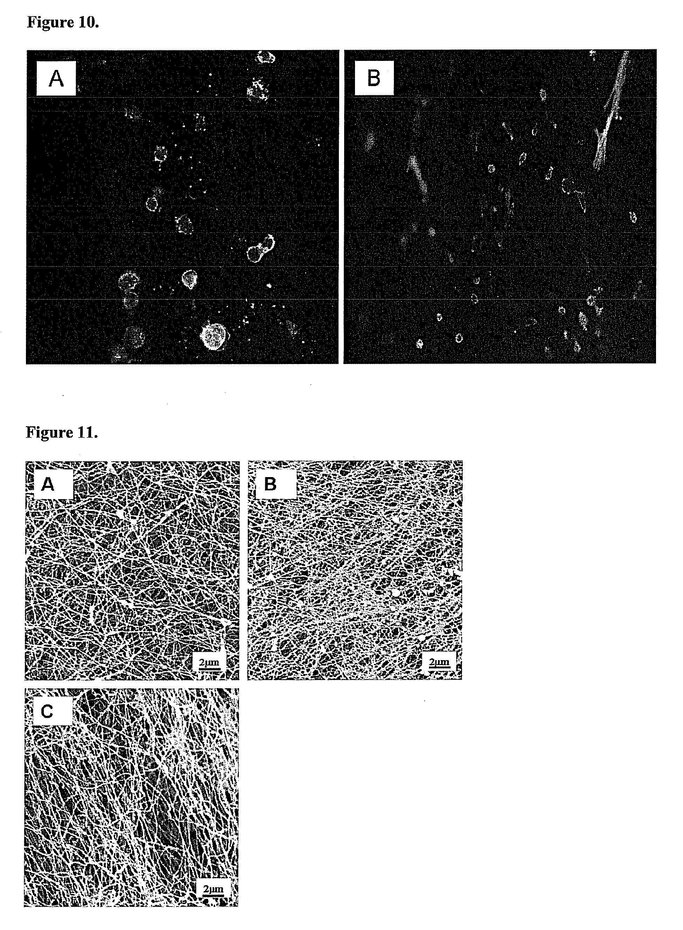

Initial cell adhesion studies showed that the adhesion of cells was faster and better on the xyloglucan-GRGDS modified cellulose than on the unmodified and xyloglucan modified cellulose. Light microscopy images show that there are a greater number of cells on the modified surface and that the extension and adhesion is more developed, See Figure 10.

EXAMPLE 12

Morphology

The morphology of cotton linters and bacterial cellulose differs in many aspects. The cotton linters are composed of fibres whose surface is covered by microfibrils. The size of the fiber is about 6$\mu$m. Bacterial cellulose on the other hand is a swollen three dimensional network consisting of nano fibrils of a size of 70-100 nm. Modifying bacterial cellulose with xyloglucan in a water phase did not alter the morphology (Figure 11C). This is not the case if modification is done in organic solvents, e.g. acetone, where the network clearly shrinks, compare Figure 11A and 11B. In order to preserve the network of BC, modification in water is preferable. A Z-scan in confocal of modified bacterial cellulose with fluorescent xyloglucan (Xyloglucan-FITC) reveals that the nano cellulose material is modified homogeneously throughout.

Conclusions

The inventors of the present invention describe a new method to modify cellulose nanofibrils with unaffected morphology of the nano fibril network. Bacterial cellulose was successfully modified with xyloglucan-GRGDS, as verified with colorimetric methods. The amount adsorbed reached a maximum of 190mg/g. The nano cellulose material is modified homogeneously throughout the material as seen by SEM and z-scan in confocal microscopy. Moreover, the modification in the water phase in comparison with organic solvents was clearly advantageous for preserving the morphology. The modification increased the wettability, which might explain the decrease or negligible amount of adsorbed protein shown by QCM-D. Initial cell studies have proven that the adhesion of endothelial cells is improved when the BC hydrogel is modified with xyloglucan-GRGDS. The increased cell adhesion was not due to non-specific adsorption of fibronectin from the culture medium, as demonstrated by QCM-D, but instead to the specific presentation of the RGD epitope by XG.

While specific embodiments of the present invention have been described in detail by the examples, it is apparent that modifications and adaptations of the present invention will occur to those skilled in the art. It is to be expressly understood, however, that such modifications and adaptations are within the scope of the present invention, as set forth by the claims.

**Claims**

1. Method for preparing an implantable material by modifying a polymeric carbohydrate material (PCM) by binding a carbohydrate linker molecule (CLM) comprising a chemical group to the PCM, wherein said chemical group confers improved biocompatibility to the PCM.

2. Method according to claim 1, wherein the CLM comprising a chemical group is prepared by a method comprising the following steps: preparing xyloglucan fragments from xyloglucan polymers; and attaching one or more chemical groups to the reducing end and/or side chains of the xyloglucan fragments, whereby the CLM comprising a chemical group useful for binding to the PCM is produced.

3.  Method according to claim 1, wherein the step of modifying the PCM is performed using the following steps:

    (a) providing a carbohydrate polymer fragment (CPF) comprising a chemical group;
    (b) bringing said CPF comprising the chemical group into contact with a soluble polymeric carbohydrate (SCP) under conditions leading to the formation of a complex consisting of said CPF comprising the chemical group, and the SCP, said CPF and SCP together forming a carbohydrate linker molecule (CLM); and
    (c) contacting said CLM with the PCM to be modified under conditions where the CLM binds to the PCM.

4.  Method according to any one of claims 1-3, wherein the step of contacting and binding the CLM comprising a chemical group to the PCM is performed under aqueous conditions.

5.  Method according to any one of claims 3-4, wherein the CPF comprising the chemical group is brought into contact with the SCP in the presence of an enzyme having transglycosylation activity that is capable of promoting the formation of the complex consisting of said CPF comprising the chemical group, and at least a part of the SCP.

6.  Method according to claim 5, wherein said enzyme having transglycosylation activity is a xyloglucan endotransglycosylase (XET, EC 2.4.1.207).

7.  Method according to any one of claims 3-6, wherein the CPF is a xyloglucan-oligosaccharide (XGO) and at least a part of the SCP is derived from xyloglucan (XG).

8.  Method according any one of claims 1-7, wherein the PCM is in the form of a cellulosic material.

9.  Method according to any one of claims 1-8, wherein the PCM is a microbial-derived cellulose, and preferably the microbial-derived cellulose is produced by the bacteria *Acetobacter xylinum*.

10. Method according to any one of claims 1-9, wherein the chemical group which confers improved biocompatibility to the PCM comprises at least one of the following: a extracellular matrix (ECM) adhesion molecule, a growth factor, a cell adhesion molecule, or an adhesion peptide fragment.

11. Method according to claim 10, wherein the adhesion peptide fragment is a: Arg-Gly-Asp (RGD) containing peptide sequence; Tyr-Ile-Gly-Ser-Arg (YIGSR) containing peptide sequence; and/or Ile-Lys-Val-Ala-Val (IKVAV) containing peptide sequence.

12. Method according to claim 11, wherein the chemical group comprises at least one RGD-containing peptide sequence.

13. Method according to claim 12, wherein the chemical group comprises at least one Gly-Arg-Gly-Asp-Ser (GRGDS) peptide sequence.

14. Implantable material for medical or surgical application prepared according to the method of any one of the claims 1-13.

15. Use of an implantable material prepared according to the method of any of the claims 1-13 for the manufacture of a scaffold for tissue engineering.

16. Use according to claim 15, for the manufacture of an artificial blood vessel, artificial skin, nerve scaffold or orthopaedic implant.

17. Use according to claim 15, for the manufacture of an artificial blood vessel.

18. Scaffold for tissue engineering comprising a material prepared according to the method of any one of the claims 1-13.

19. Scaffold according to claim 18, wherein the scaffold for tissue engineering has been pre-seeded with cells *in vitro.*

20. Artificial blood vessel comprising a material prepared according to the method of any one of the claims 1-13.

21. Artificial blood vessel according to claim 20, wherein the artificial blood vessel has been pre-seeded with cells *in vitro.*

**22.** Method of in vivo tissue replacement and/or regeneration comprising the following steps:

    a) providing a scaffold for tissue engineering comprising implantable material prepared according to any one of the claims 1-13; and
    b) implanting said material into a suitable implantation site of a subject in need thereof.

**23.** Method according to claim 22, wherein the scaffold for tissue engineering is pre-seeded with cells *in vitro* before the step of implanting said scaffold.

**24.** Method according to any one of claims 22-23, wherein the scaffold for tissue engineering is an artificial blood vessel.

**Figure 1.**

**Figure 2.**

**Figure 3.**

**Figure 4.**

**Figure 5.**

**Figure 6.**

**Figure 7.**

**Figure 8.**

**Figure 9.**

**Figure 10.**

**Figure 11.**

**European Patent**
**Office**

**PARTIAL EUROPEAN SEARCH REPORT**    Application Number

which under Rule 45 of the European Patent Convention EP 07 10 3055
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEO S-J ET AL: "Alginate microcapsules prepared with xyloglucan as a synthetic extracellular matrix for hepatocyte attachment" BIOMATERIALS, ELSEVIER SCIENCE PUBLISHERS BV., BARKING, GB, vol. 26, no. 17, June 2005 (2005-06), pages 3607-3615, XP004696109 ISSN: 0142-9612 * the whole document * ----- | 1,14,15, 18,22 | INV. A61L27/20 A61L27/54 A61L31/04 A61L31/16 A61L33/08 |
| Y | YANG YING ET AL: "Biodegradable scaffolds--delivery systems for cell therapies." EXPERT OPINION ON BIOLOGICAL THERAPY MAY 2006, vol. 6, no. 5, May 2006 (2006-05), pages 485-498, XP009088220 ISSN: 1744-7682 * the whole document * ----- -/-- | 1-24 | |

TECHNICAL FIELDS
SEARCHED    (IPC)

A61L

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 14 August 2007 | Hars, Jesko |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C07)

**EP 1 961 432 A1**

## DOCUMENTS CONSIDERED TO BE RELEVANT

| Category | Citation of document with indication, where appropriate, of relevant passages | Relevant to claim |
|---|---|---|
| Y | BRUMER HARRY III ET AL: "Activation of crystalline cellulose surfaces through the chemoenzymatic modification of xyloglucan" JOURNAL OF THE AMERICAN CHEMICAL SOCIETY, vol. 126, no. 18, 12 May 2004 (2004-05-12), pages 5715-5721, XP002446748 ISSN: 0002-7863 * the whole document * ----- | 1-24 |
| Y | ZHOU QI ET AL: "XYLOGLUCAN AND XYLOGLUCAN ENDO-TRANSGLYCOSYLASES (XET): TOOLS FOR EX VIVO CELLULOSE SURFACE MODIFICATION" BIOCATALYSIS AND BIOTRANSFORMATION, HARWOOD ACADEMIC PUBL., BASEL, CH, vol. 24, no. 1-2, January 2006 (2006-01), pages 107-120, XP009079822 ISSN: 1024-2422 * the whole document * ----- | 1-24 |
| D,Y | WO 2004/094646 A (UNIV GEORGIA [US]; ALBERSHEIM PETER [US]; DARVILL ALAN [US]; HEISS CHR) 4 November 2004 (2004-11-04) * the whole document * ----- | 1-24 |
| Y | SVENSSON A ET AL: "Bacterial cellulose as a potential scaffold for tissue engineering of cartilage" BIOMATERIALS, ELSEVIER SCIENCE PUBLISHERS BV., BARKING, GB, vol. 26, no. 4, February 2005 (2005-02), pages 419-431, XP004522660 ISSN: 0142-9612 * the whole document * ----- | 1-24 |

-/--

**CLASSIFICATION OF THE APPLICATION (IPC)**

**TECHNICAL FIELDS SEARCHED (IPC)**

EPO FORM 1503 03.82 (P04C10)

European Patent
Office

**PARTIAL EUROPEAN SEARCH REPORT**

Application Number

EP 07 10 3055

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | BACKDAHL ET AL: "Mechanical properties of bacterial cellulose and interactions with smooth muscle cells" BIOMATERIALS, ELSEVIER SCIENCE PUBLISHERS BV., BARKING, GB, vol. 27, no. 9, March 2006 (2006-03), pages 2141-2149, XP005204073 ISSN: 0142-9612 * the whole document * ----- | 1-24 | |
| D,Y | BODIN AASE ET AL: "Influence of cultivation conditions on mechanical and morphological properties of bacterial cellulose tubes" BIOTECHNOLOGY AND BIOENGINEERING, vol. 97, no. 2, 29 June 2006 (2006-06-29), pages 425-434, XP002446749 ISSN: 0006-3592 * the whole document * ----- | 1-24 | TECHNICAL FIELDS SEARCHED (IPC) |

EPO FORM 1503 03.82 (P04C10)

European Patent
Office

INCOMPLETE SEARCH
SHEET C

Application Number

EP 07 10 3055

Although claims 22-24 are directed to a method of treatment of the human/animal body (Article 52(4) EPC), the search has been carried out and based on the alleged effects of the compound/composition.

-----

Reason for the limitation of the search (non-patentable invention(s)):

Article 52 (4) EPC - Method for treatment of the human or animal body by therapy
Article 52 (4) EPC - Method for treatment of the human or animal body by surgery

-----

Further limitation of the search

Claim(s) searched incompletely:
        1-24

Reason for the limitation of the search:

Technical features and their related technical effects:
a) the adsorption of XG (xyloglucan) onto cellulose imparts strength,
b) the adsorption of XG, which is carried out at mild reaction conditions, maintains the cellulose fiber network, as compared to using organic synthesis substituting the cellulose directly,
c) the use of XGOs (xyloglucan oligosaccharides) renders the addition of GRGDS selective, compared to rather unreactive XG,
d) the use of XGOs as a linker allows the easy enzymatic covalent binding of the XGO-GRGDS to the XG,
e) GRGDS increases adhesion and proliferation of endothelial cells and therefore blood compatibility,
f) the use of BC instead of wood cellulose improves the biocompatibility.

It is noted that several of the above named technical features are not present in the independent claims. The subject-matter referred to in these claims consequently also lacks the technical effects associated. It is further noted that the claims introduce the essential components of the invention, ie cellulose, XG, XGO and peptide, piecemeal in the dependent claims. Since these four entities, or at least cellulose, XG and XGO, form a true combination enabling the biocompatible functionalisation of cellulose and subsequent use as tissue scaffold, making any of them optional results in the loss of the combination aspect, ie the loss of the gist of the invention.
In other words, the independent claims do not comprise all features essential to the invention (Art. 84 EPC).
Examination and search is limited to a hypothetical claim covering cellulose, XG, XGO and a biocompatible factor covalently attached to the XGO.

## ANNEX TO THE EUROPEAN SEARCH REPORT
## ON EUROPEAN PATENT APPLICATION NO.

EP 07 10 3055

This annex lists the patent family members relating to the patent documents cited in the above-mentioned European search report.
The members are as contained in the European Patent Office EDP file on
The European Patent Office is in no way liable for these particulars which are merely given for the purpose of information.

14-08-2007

| Patent document cited in search report | Publication date | Patent family member(s) | Publication date |
|---|---|---|---|
| WO 2004094646 A | 04-11-2004 | CA 2523263 A1 | 04-11-2004 |
| | | EP 1627070 A1 | 22-02-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6800753 B
- WO 03033813 A
- WO 2004094646 A1
- JP 8126697 A
- EP 186495 A
- JP 3165774 A
- JP 63205109 A
- JP 3272772 A
- EP 396344 A2
- EP 0396344 A

### Non-patent literature cited in the description

- **FRY et al.** *Physiologia Plantarum,* 1993, vol. 89, 1-3
- **SCHRODER, R. et al.** *PLANTA,* October 2006, vol. 224 (5), 1091-1102
- **SCHRODER R. et al.** *PLANTA,* August 2004, vol. 219 (4), 590-600
- **HERSEL et al.** *Biomaterials,* 2003, vol. 24, 4385-4415
- **GRAF et al.** *Biochemistry,* 1987, vol. 26, 6896-900
- **TASHIRO et al.** *J. Biol. Chem,* 1989, vol. 264, 16174-182
- **BODIN et al.** Influence of cultivation conditions on mechanical and morphological properties of bacterial cellulose tubes. *Biotechnol Bioeng,* 29 December 2006
- **KONTTURI et al.** *Langmuir,* 2003, vol. 19, 5735-5741
- **GREFFE, L. et al.** *Glycobiology,* 2005, vol. 15 (4), 437-445
- **ENGFELDT et al.** *Chembiochem: a European journal of chemical biology,* 2005, vol. 6 (6), 1043-50
- **KOOIMAN et al.** *Recueil des Travaux Chimiques des Pays-Bas et de la Belgique,* 1960, vol. 79, 675-678
- **OUGIYA et al.** *Bioscience, Biotechnology, and Biochemistry,* 1998, vol. 62, 1714-1719
- **BRUMER, H. et al.** *Journal of the American Chemical Society,* 2004, vol. 126 (18), 5715-5721